## Europäisches Patentamt
## European Patent Office
## Office européen des brevets

(11) Numéro de publication: **0 132 170**
**B1**

# FASCICULE DE BREVET EUROPÉEN

(45) Date de publication du fascicule du brevet:
**25.01.89**

(21) Numéro de dépôt: **84401217.9**

(22) Date de dépôt: **14.06.84**

(51) Int. Cl.⁴: **G 01 N 33/53,** G 01 N 33/543, G 01 N 33/546, G 01 N 33/577

(54) **Procédé de détection d'un oligomère dans un milieu liquide contenant des monomères correspondants, dispositif pour la mise en oeuvre de ce procédé, et application notamment à la recherche d'allergènes.**

(30) Priorité: **14.06.83 FR 8309806**

(43) Date de publication de la demande:
**23.01.85 Bulletin 85/4**

(45) Mention de la délivrance du brevet:
**25.01.89 Bulletin 89/4**

(84) Etats contractants désignés:
**AT BE CH DE GB IT LI LU NL SE**

(56) Documents cité:
**EP-A-0 051 985**
**EP-A-0 098 590**
**FR-A-2 235 368**
**FR-A-2 487 984**
**GB-A-1 506 133**
**GB-A-2 026 691**
**GB-A-2 043 888**
**US-A-4 332 783**

**CHEMICAL ABSTRACTS, vol. 94, no. 11, 16 mars 1981, page 537, no. 81881d, Columbus, Ohio, US; K.A. KELLY et al.: "The development of a radioallergosorbent test (RAST) for murine IgE antibodies"**
**CHEMICAL ABSTRACTS, vol. 98, no. 25, 20 juin 1983, page 414, no. 213869a, Columbus, Ohio, US; D.H. CONRAD et al.: "Properties of two monoclonal antibodies directed against the Fc and Fab regions**

(73) Titulaire: **BIO MERIEUX Société Anonyme ( anciennement dénommée BME ), Marcy- l'Etoile, F-69752 Charbonnières- les- Bains (FR)**

(72) Inventeur: **Mandrand, Bernard, 21, petite rue de la Doua, F-69100 Villeurbanne (FR)**
Inventeur: **Bremond, Florence, 30, rue Soeur Bouvier, F-69005 Lyon (FR)**
Inventeur: **Trouyez, Gérard, 30, rue Chazière, F-69004 Lyon (FR)**

(74) Mandataire: **Nony, Michel, Cabinet Nony 29, rue Cambacérès, F-75008 Paris (FR)**

(56) Documents cité: (suite)
**of rat IgE"**

**Description**

La présente invention a pour objet un procédé de détection d'un oligomère dans un milieu liquide contenant des monomères correspondants, ainsi qu'un dispositif destiné à la mise en oeuvre de ce procédé et l'application de ce procédé, notamment à la détection de substances allergènes.

Le procédé de l'invention est en particulier applicable aux molécules ou macro-molécules monomères ou oligomères d'origine biologique.

On sait qu'il existe de nombreux dispositifs destinés à repérer la présence d'une molécule biologique déterminée (antigène, anticorps, etc...) dans un milieu liquide, par exemple à l'aide de réactifs présentés sous forme de billes de polystyrène ou sous forme de plaques de microtitration.

On connaissait déjà, par le brevet US 4.332.783 un procédé de détection d'un complexe immun à l'aide d'un premier réactif, fixé sur un support solide, spécifique de l'antigène dudit complexe, et d'un second réactif, spécifique de l'anticorps, ou vice versa. Un tel procédé nécessite la préparation d'un réactif particulier, pour chaque antigène donné. Le procédé de la présente invention apporte des simplifications notables par rapport à ce procédé connu, car il permet, avec l'utilisation d'un seul même réactif, la détection de complexes immuns formés avec des antigènes différents, comme cela sera exposé ci-après.

L'invention a pour objet un procédé permettant de détecter un oligomère contenant au moins deux molécules possédant chacune un même site récepteur non répétitif, dans un milieu liquide contenant ou susceptible de contenir une solution ou dispersion de ladite molécule à l'état de monomères, ladite solution ou dispersion contenant aussi, ou étant susceptible de contenir, ledit oligomère. Ce procédé est caractérisé par le fait que l'on met en contact avec ladite solution ou dispersion un support solide sur lequel sont fixées des molécules liantes douées d'une affinité spécifique pour ledit site récepteur non répétitif, que l'on ajoute à ladite solution ou dispersion une solution ou dispersion de ladite molécule liante couplée à un agent traceur, et que l'on détermine qualitativement et/ou quantitativement la présence éventuelle dudit agent traceur fixé sur ledit support solide.

Parmi les oligomères qu'il est possible de repérer grâce au procédé de l'invention, on citera notamment les complexes immuns tels que les complexes du type anticorps/antigène/anticorps Dans ce cas, ladite molécule liante est un second anticorps spécifique dirigé contre un site non répétitif du premier anticorps.

Bien entendu, le milieu liquide dans lequel on effectue la détection est un milieu ne dissociant pas lesdits oligomères. Dans le cas de molécules biologiques, il s'agit des solutions tampons utilisées de façon usuelle.

La molécule liante peut être fixée sur le support solide par tout moyen connu pour la fixation de ces molécules, par exemple par adsorption, par liaison covalente ou non, par exemple par couplage chimique avec un agent de couplage bi-fonctionnel, par affinité imunochimique, etc...

Le support solide peut être réalisé avec tout matériau solide biologique (globules rouges) ou synthétique doué de propriétés adsorbantes ou capable de fixer un agent de couplage. Ces matériaux sont connus et décrits dans la littérature. Parmi les matériaux solides capables de fixer par exemple des anticorps par adsorption, on citera notamment le polystyrène, le polypropylène. etc... Parmi les matériaux utilisables pour fixer lesdites molécules liantes par covalence à l'aide d'un agent de couplage, on cite notamment le dextran, la cellulose, et leurs dérivés aminés tels que la diéthylamino-cellulose ou le diéthylamino-dextran.

Le support peut se présenter par exemple sous forme de disques, de cubes, de baguettes, de billes ou de plaques, par exemple de plaques de microtitration. Le support peut également être un latex en suspension.

Les agents de couplage permettant de fixer par covalence les molécules liantes sur le support solide sont par exemple des dérivés carboxyliques, des dérivés bifonctionnels tels que les dialdéhydes (glutaraldéhyde), des diisocyanates (toluéne-diisocyanate), des quinones (benzo-quinone), etc...

Quand l'oligomère à repérer est un complexe immun formé par un premier anticorps avec l'antigène correspondant, la molécule liante est un second anticorps spécifique dirigé contre un site non répétitif dudit premier anticorps. De préférence, ledit second anticorps spécifique est dirigé contre un site non répétitif du fragment Fc dudit premier anticorps.

Ledit premier anticorps est par exemple une immunoglobuline IgE, IgD, IgG ou IgA, ou appartient à une sous-classe de ces immunoglobulines. De préférence, le second anticorps est un anticorps monoclonal ou au moins un anticorps hautement spécifique purifié par exemple par chromatographie d'affinité.

On peut également mettre en oeuvre le procédé de l'invention en utilisant comme molécule liante une lectine. On sait que les lectines ont une affinité pour des sucres constitutifs de certains sites des molécules d'immunoglobuline ou pour des sucres présents sur d'autres molécules biologique ou présents sur la membrane externe de certaines cellules.

Dans le procédé de l'invention, ledit oligomère peut être formé in situ par addition, à un milieu liquide contenant ou susceptible de contenir lesdits monomères, d'une substance dimérisante ou oligomérisante spécifique pour lesdits monomères ou pour une sous-classe desdits monomères. Dans ce cas, le procédé de l'invention est caractérisé par le fait que l'on ajoute audit milieu liquide contenant lesdits monomères, une substance dimérisante ou oligomérisante spécifique pour lesdits monomères ou pour une sous-classe desdits monomères que ledit milieu liquide est susceptible de contenir, de façon à former in situ ledit oligomère et à détecter la présence éventuelle dudit oligomère, et en conséquence la présence du monomère pour lequel ladite substance dimérisante ou oligomérisante est spécifique.

La susbtance dimérisante ou oligomérisante est par exemple un antigène, et ledit monomère est bien entendu dans ce cas l'anticorps correspondant. Ce procédé permet donc de déterminer s'il existe, dans une

2

**EP 0 132 170 B1**

solution contenant des anticorps, des anticorps spécifiques de l'antigène ajouté. En particulier lorsque l'antigène est un allergène, on peut déterminer si le milieu liquide étudié contient des IgE monomères dirigés contre ledit allergène.

Ce dernier procédé est particulièrement intéressant car il permet d'ajouter la substance dimérisante ou oligomèrisante sous une forme liquide.

L'agent traceur est tout agent qui peut être couplé avec la molécule liante pour donner un conjugué dont il est facile de repérer la présence sur le support solide après lavage convenable. Les agents traceurs sont bien connus et sont constitués par exemple par des produits fluorescents (fluorescéine), des produits isotopiques marqués et des enzymes donnant des réactions colorées ou fluorescentes.

Dans un autre mode de réalisation particulier, le procédé de l'invention est caractérisé par le fait que ladite molécule liante est fixée sur de petites particules solides individuelles capables de se maintenir en suspension dans ledit milieu liquide, que lesdites particules solides jouent à la fois le rôle dudit support solide et dudit traceur, et que l'on repère une éventuelle agglutination avec des moyens appropriés (par exemple par l'observation visuelle directe, ou à l'aide d'un appareil optique).

Les particules qui sont utilisables sont notamment des particules de latex.

L'invention a également pour objet un dispositif destiné à la mise en oeuvre du procédé, caractérisé par le fait qu'il comprend une ou plusieurs unités dudit support solide sur lequel est fixée ladite molécule liante, un ou plusieurs conteneurs contenant ladite molécule liante couplée à un agent traceur, éventuellement des solutions d'antigènes ou d'allergènes, des tampons de dilution, d'incubation et de lavage, et éventuellement un mode d'emploi, le tout étant réuni dans un même emballage à plusieurs compartiments.

L'invention va maintenant être décrite plus en détail en faisant référence à des supports solides sur lesquels sont fixés des anticorps monoclonaux anti-IgE qui sont dirigés contre un site spécifique des molécules d'IgE; on sait que ces dernières sont présentes en quantité importante dans l'organisme dans le cas de réactions allergiques. Il faut remarquer que c'est uniquement pour faciliter la compréhension de l'exposé qu'on a décrit plus spécialement le recherche des complexes formés avec des anticorps du type IgE. Les spécialistes comprendront que le procédé décrit n'est nullement limité à ces anticorps particuliers, et la suite de la description ne doit donc en aucun cas être considérée comme limitative à cet égard.

Sur le plan clinique, la réaction allergique est caractérisée notamment par l'élaboration dans l'organisme humain d'anticorps de classe IgE, spécifiques d'une substance d'origine externe, l'allergène. L'allergène est une substance haptènique ou antigénique susceptible de provoquer la dégranulation des basophiles humains par pontage des IgE fixées sur ces cellules par leur fragment Fc et sur l'allergène par leur fragment Fab.

Une proportion assez forte des IgE de sujets allergiques est spécifique d'un ou plusieurs allergènes. Il existe également des complexes immuns circulants du type IgE-Allergène-IgE.

La détermination de l'allergène responsable a un intérêt médical certain, soit préventif pour éviter au sujet allergique le contact avec l'agent responsable, soit curatif pour mettre en oeuvre une thérapeutique de désensibilisation.

Depuis quelques années, des méthodes ont été proposées pour mettre en évidence in vitro une fixation spécifique des IgE humaines. Parmi ces méthodes, il faut citer le test de dégranulation des basophiles humains, le test radioimmunologique RAST et son équivalent immuno-enzymatique.

Les méthodes les plus proches de l'invention utilisent un allergène fixé sur une phase solide. Le sérum est mis en contact avec cet allergène fixé et la réaction spécifique prend place sur le support. L'IgE est ensuite révélée par un anticorps spécifique.

Dans certaines méthodes publiées récemment, notamment dans "The Journal of Immunological Methods", l'allergène est marqué par un atome radioactif et l'anticorps IgE est fixé sur une bille (J. of I. Methods volume 58, n° 1 - 2, 1983 - pp 49 - 57).

Aucune méthode proposée ne permet à la fois de détecter des complexes immuns circulants et d'en analyser la composition à l'aide d'un anticorps monoclonal.

Le système décrit ici permet de rechercher les complexes immuns formés avec des, IgE et de déterminer la nature de l'allergène impliqué dans une réaction allergique.

Le réactif de l'invention utilise un seul type d'anticorps monoclonal spécifique d'un site non répétitif de la molécule d'IgE, ledit site étant de préférence situé sur le fragment Fc de la molécule.

D'une part, ces anticorps monoclonaux identiques sont fixés sur une phase solide soit par covalence, soit par adsorption passive. La phase solide peut être en suspension, séparable par centrifugation ou par force magnetique. Elle sera de préférence en polymère organique de type polystyrène ou polychlorure de vinyle, et présentée sous forme de bille, de tube, de plaque de microtitration ou de sous-unités d'une telle plaque.

D'autre part, des anticorps monoclonaux identiques aux premiers sont marqués par un composé radioactif, une enzyme, une molécule fluorescente ou toute autre molécule utilisée comme élément de détection et de quantification des réactions immunochimiques.

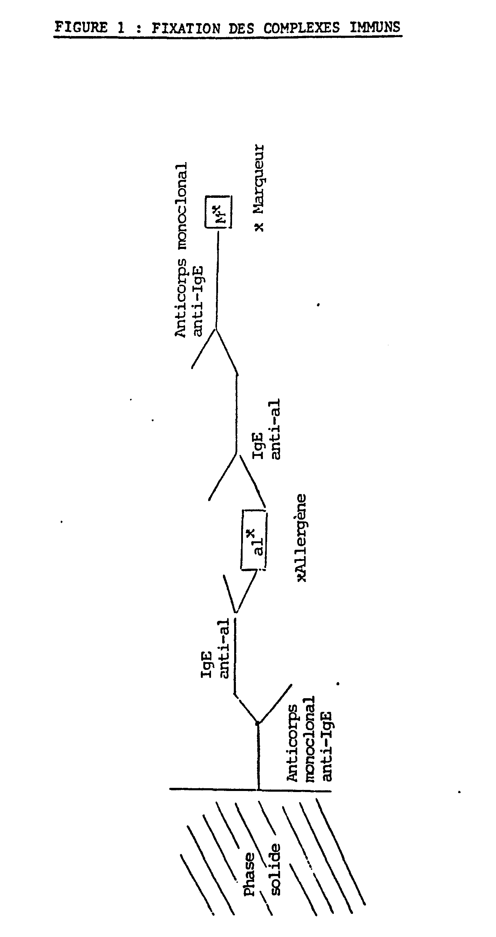

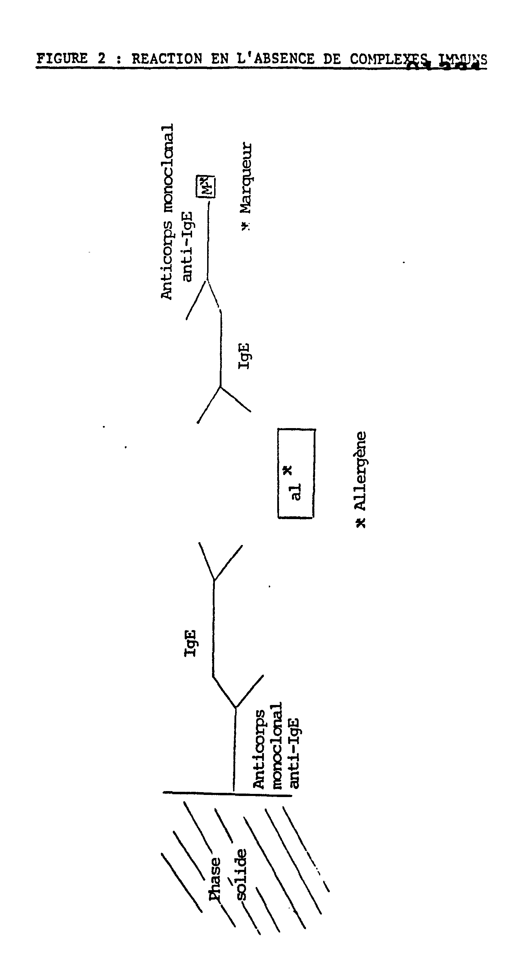

Les complexes immuns du type IgE-Allergène-IgE sont pris en "sandwich" entre les anticorps fixés et les anticorps marqués (figure 1). La quantification est réalisée par le système de marquage utilisé, préférentiellement après lavage de la phase solide. Les molécules d'IgE non complexées, non dimérisées, se fixent soit sur la phase solide, soit sur l'anticorps marqué, mais en l'absence de l'allergène correspondant à leur spécificité, elles ne réaliseront pas le sandwich (figure 2).

Le système peut être utilisé de deux manières différentes, soit pour repérer et quantifier les complexes

EP 0 132 170 B1

immuns à IgE existant dans le sérum (en cas de crise allergique par exemple, ou pour suivre l'évolution d'une désensibilisation) soit après incubation in vitro et obtention des complexes IgE-Allergènes-IgE, en utilisant des allergènes, présentés sous forme liquide, que l'on ajoute pour former ces complexes, in situ, dans le cas où des IgE spécifiques de l'allergène étudié sont présents.

L'intérêt de l'invention réside notamment dans ces deux possibilités. La détection des complexes immuns est, en effet, plus probablement significative d'une situation allergique que la détermination d'un taux élevé d'IgE totales.

Par ailleurs, l'utilisation d'allergènes non fixés, dans un test spécifique in vitro, réduit considérablement la mise au point d'un nouveau dosage, par rapport aux systèmes précédents, du fait de la suppression de l'étape de fixation ou de marquage qui impose de résoudre des problèmes de couplage chimique propres à chaque système allergénique.

Le nouveau procédé décrit ici utilise les solutions allergéniques telles qu'elles sont présentées aux cliniciens pour la désensibilisation. Ce système utilise en outre une seule phase solide, revêtue d'un type unique d'anticorps, et une seule solution d'anticorps marqué.

L'anticorps utilisé doit être rigoureusement monospecifique. Il ne doit réagir qu'avec un seul site non répétitif de la molécule d'IgE. Deux anticorps agissant sur des sites différents, mais mutuellement exclusifs, peuvent être toutefois utilisables.

Un analogue structural des récepteurs Fc des basophiles humains est utilisable également.

Le système peut être très facilement adapté non seulement aux techniques radioimmunologiques, immunoenzymatiques et immunofluorescentes, mais aussi à la technique d'agglutination de particules (de latex par exemple). Dans ce cas, un seul réactif est nécessaire ; il contient des particules de latex revêtues de l'anticorps monoclonal anti-IgE. Les particules de latex jouent à la fois le rôle de support solide et d'agent traceur générateur d'un signal (ici l'agglutination). Une réaction positive se traduit par une agglutination visible sur lame ou détectée soit par comptage de particules, soit par néphélémétrie, soit par toute autre méthode sensible de mesure des complexes du type Latex-anti-IgE / IgE-Allergène - IgE / anti - IgE - Latex.

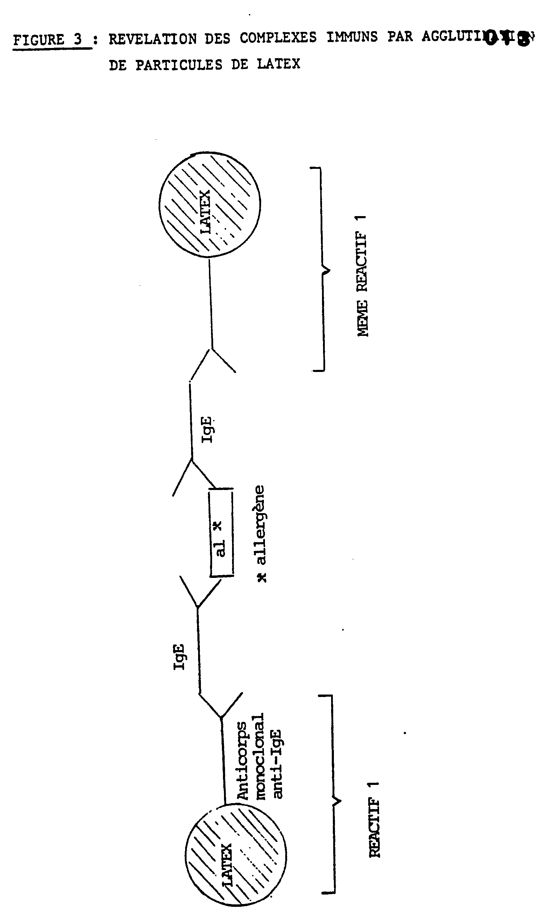

Ce système, plus sensible aux interférences que les méthodes utilisant une étape de lavage, a cependant l'intérêt de n'utiliser qu'un seul réactif employant l'anticorps monoclonal, au lieu de deux comme dans le cas précédent (figure 3).

D'autres procédés d'agglutination, utilisant par exemple des sels métalliques, peuvent également être utilisés, par exemple ceux utilisant des sels d'or pour constituer un réseau colloïdal.

Le système présenté est applicable non seulement à l'allergie, mais également à toute recherche de complexes immuns où l'on veut établir la spécificité de l'anticorps impliqué.

Par exemple, pour la recherche des IgG spécifiques, le système décrit précédemment est complètement transposable. L'anticorps anti-IgE est remplacé par un anticorps monoclonal anti-IgG, l'allergène devient un antigène de type bactérien, viral ou parasitaire. La détection peut porter sur des complexes immuns circulants, ou sur des complexes formés in vitro dans le but de déterminer l'antigène responsable de l'infection. Dans ce dernier cas, il est utile soit d'éliminer par les méthodes connues, avant d'ajouter l'antigène, les dimères ou oligomères éventuellement présents, soit de procéder par comparaison avec une solution témoin à laquelle on n'ajoute pas d'antigène.

Pour les IgM, le même système est réalisable à condition soit de disposer d'un anticorps monoclonal ne reconnaissant qu'un seul cycle sur la molécule pentamérique IgM, soit de dissocier préalablement les sous-unités monomères par un agent réducteur tel que le mercapto-éthanol.

Un système tout aussi efficace utilise des lectines en lieu et place des anticorps monoclonaux. La spécificité de ces lectines est dirigée contre des sucres constitutifs des molécules d'immunoglobulines. Chaque fois qu'un site unique de fixation (ou des sites mutuellement exclusifs) sera impliqué, le procédé sera applicable.

Les exemples suivants illustrent l'invention sans toutefois la limiter.

## EXEMPLE 1. Recherche des complexes immuns à IgE

La réaction utilise comme support une plaque de microtitration en polystyrène spécialement traitée pour donner un niveau de fixation élevé des anticorps. Tout autre support utilisé en technique immunoenzymatique (EIA), tel que tubes, billes, barettes etc... pourrait également convenir.

L'anticorps, préférablement dilué en tampon carbonate de faible force ionique, à pH neutre ou alcalin, se fixe pendant une nuit. La solution d'anticorps déposée dans chaque puits contient 5 µg/ml d'anticorps monoclonal obtenu de souris ou de rat.

L'anticorps est éliminé et la phase solide lavée avec un tampon salin à pH neutre contenant un agent tensioactif, de type Tween 20 par exemple. A ce stade, la plaque peut être séchée sous vide et conservée en sachet étanche en présence de déshydratant. La solution d'anticorps peut également être conservée sur la plaque quelques semaines si l'évaporation est évitée par un film adhésif.

Les sérums à analyser sont dilués et incubés sur la plaque en tampon neutre contenant des protéines, préférablement de l'ascite de souris diluée en présence d'albumine bovine pour éviter les interférences sériques. Après une incubation de quelques minutes à quelques heures, suivant l'affinité de l'anticorps la plaque est lavée et on répartit un volume égal (10 à 200 µl) de conjugué formé par couplage d'un anticorps

4

monoclonal identique au premier avec une enzyme (préférentiellement la β-Galactosidase, la phosphatase alcaline ou la peroxydase).

Après incubation du conjugué pendant quelques minutes à quelques heures, la plaque est lavée et le substrat de l'enzyme (coloré ou fluorescent) est réparti dans les puits utilisés de la plaque. Après incubation, le substrat est bloqué par une solution inhibitrice de l'activité enzymatique et la densité optique ou la fluorescence est mesurée.

Les puits montrant une activité enzymatique traduisent la présence de complexes à IgE ou d'IgE polymérisées. Une courbe d'étalonnage peut être réalisée avec un sérum contenant un taux connu de complexes afin de quantifier exactement les complexes sériques des échantillons.

Si l'anticorps monoclonal est de très forte affinité, et s'il est utilisé très concentré, l'incubation des sérums et du conjugué anticorps-enzyme peut être simultanée. Les volumes et les temps sont ceux utilisés généralement en technique immunoenzymatique. L'enzyme peut être remplacée par de l'iode radioactive ou par un composé fluorescent en adoptant alors un moyen de détection approprié.

## EXEMPLE 2. Détermination de l'allergène spécifique

La réaction utilise les éléments précédents support ayant fixé l'anticorps, et conjugué anticorps-enzyme:

Après réalisation in vitro de complexes IgE-Allergène-IgE par addition de différentes concentrations de l'allergène supposé, le complexe est mis à incuber comme précédemment.

Des concentrations élevées d'allergène dissocient les complexes pré-existants, alors qu'une concentration optimale permet de réaliser un maximum de sites de fixation polyvalents pour l'anticorps monoclonal.

Il s'agit là de phénomènes bien connus et la recherche d'une concentration optimale peut être effectuée par de simples expériences de routine avec diverses dilutions d'allergène. Le mélange IgE-allergène est réalisé en tube et laissé incuber pendant quelques minutes à quelques heures à une température variant entre 4°C et 56°C.

On réalisera de préférence des dilutions d'allergènes à partir des solutions utilisées pour la désensibilisation par les allergologues. Les dilutions types iront de quelques mg d'allergène par ml à quelques pg/ml.

Les mélanges sérum + allergène sont répartis sur des plaques revêtues d'anticorps, et incubés quelques minutes à quelques heures. Le support est lavé et le conjugué réparti. Après incubation du conjugué et lavage, le substrat est incubé, puis bloqué et le signal est mesuré.

Les résultats montrent qu'une forte concentration d'allergène spécifique inhibe la réaction de fixation en "sandwich" de l'anticorps fixé sur la phase solide et de l'anticorps marqué. Une concentration optimale au contraire se traduit par un signal mesuré très élevé.

La quantification de ce signal peut être faite en relation avec un couple sérum-allergène connu.

La réaction peut être simplifiée en présentant en même temps sur la phase solide revêtue d'anticorps, en une seule incubation, le sérum contenant les IgE spécifiques, l'allergène et le conjugué anti-IgE-enzyme. Deux dilutions d'allergènes sont suffisantes en essais de routine pour déterminer la spécificité de l'IgE.

Si l'allergène utilisé ne correspond pas au sérum, les deux dilutions donneront des signaux identiques. Si l'allergène est celui contre lequel sont dirigées les IgE du sérum, le signal mesuré sera plus élevé pour la dilution la plus faible de l'allergène, si celle-ci a été correctement choisie. Des dilutions trop faibles d'allergène donnent en effet un résultat identique à celui du sérum seul.

Les réactifs (à savoir anticorps fixé, tampon de dilution, conjugué ou allergène) peuvent en outre être présentés séchés ou lyophilisés sur le support.

Dans une réaction immunoenzymatique, le substrat seul serait à ajouter après incubation du système (anticorps fixé / IgE - Allergène IgE / Conjugué anticorps-enzyme) et lavage.

Dans une réaction radioimmunologique, immunfluorescente, le lavage précéderait immédiatement la lecture directe.

## Applications

Ces applications utilisent des anticorps monoclonaux.

Ces anticorps sont obtenus par production de cellules hybrides de souris. L'immunisation a été réalisée avec des IgE humaines et l'anticorps sélectionné est dirigé contre le fragment Fc de la molécule d'IgE. L'anticorps à fixer est incubé une nuit à 4°C en tampon carbonate 50 mM pH 9,6 à raison de 200 µl par puits sur une plaque de microtitration de type Immuno I fabriquée par la Société NUNC (Roskilde - DK). L'anticorps est à une concentration finale de 5 g/ml.

Pendant le même temps, un sérum de patient non allergique aux graminées, et un sérum de patient allergique, sont dilués au 1/10 en tampon protéique et mis à incuber avec une solution allergénique contenant 10% d'extrait de graminées et dilués entre 40 et 400 000 fois.

Les mélanges allergènes-sérum sont répartis, soit 200 µl par puits de la plaque. Après deux heures d'incubation à 22°C et lavage, le conjugué est réparti et incubé 1 heure à 22°C.

Le conjugué a été réalisé par couplage au glutaraldéhyde de l'anticorps monoclonal et de la galactosidase d'E. Coli selon la technique d'Avraméas.

Après lavage, le substrat, orthonitrophényl-β-D-galactosidase est réparti et incubé pendant un temps variable suivant la qualité de l'anticorps. Le volume est ici encore de 200 µl. Après coloration, la réaction enzymatique est arrêtée par 100 µl d'une solution de glycine/NaOH pH 10,4.

La lecture des densités optiques est réalisée directement sur la plaque 405 nm avec l'appareil LOV 1 Biomérieux. Le zéro de l'appareil est effectué sur une solution contenant le substrat seul.

Les résultats fournis sont en densité optique lue directement après blocage par le tampon glycine NaOH.

**Application I - Utilisations de l'anticorps XB6-16**

On opère selon la même technique que précédemment pour préparer les réactifs.

L'anticorps utilisé est fourni par l'Institut Pasteur Produciton sous la référence XB6-16.

Le tampon de dilution du mélange allergène-sérum est un tampon PBS-Tween contenant 17 % de liquide d'ascite de souris obtenu par inoculation d'une souche sarcomateuse.

Le sérum négatif utilisé provient d'un patient connu non allergique. Le sérum positif a été prélevé sur un patient allergique aux graminées.

L'allergène employé est un extrait de dactyle (graminées) produit par le laboratoire des Stallergènes (160 quai de Polangis, Nogent sur Marne, France). Le substrat a été incubé une heure à 22° C.

**Les résultats** (densités optiques) sont résumés dans le tableau I.

**TABLEAU I**

| | Témoin sans allergène | Dilution de la solution d'allergène | | | |
|---|---|---|---|---|---|
| | | 1/40 | 1/400 | 1/4000 | 1/40 000 |
| Diluant seul | | 0,001 | 0,004 | 0,002 | 0,001 |
| Sérum négatif | 0,032 | 0,057 | 0,074 | 0,064 | 0,049 |
| Sérum positif | 0,811 | 0,103 | 0,749 | 0,951 | 0,730 |

Le sérum négatif seul ou en combinaison avec l'allergène ne donne aucune réponse significative. Le sérum positif seul donne une densité optique élevée traduisant l'importance des complexes immuns circulants présents dans ce sérum.

La dilution 1/40 donne une densité optique faible du fait de la dissociation des complexes par l'excès d'allergène.

La dilution 1/4000 donne ici le maximum de réponse positive. Entre 1/40 et 1/4000 le densité optique est multipliée par 9 ce qui assure une évaluation très facile du phénomène allergique.

Le même test a été repris avec une deuxième préparation des mêmes réactifs. Les résultats du tableau II montrent pour le sérum positif seul des résultats comparables à ceux du tableau I, avec toutefois une réaction maintenue élevée pour une dilution d'allergène 1/400 000.

**TABLEAU II**

| | Témoin sans allergène | Dilution de l'allergène | | | | |
|---|---|---|---|---|---|---|
| | | 1/40 | 1/400 | 1/4000 | 1/40000 | 1/400000 |
| Sérum positif | 0,873 | 0,232 | 0,729 | 1,119 | 1,178 | 1,131 |

Les réactions entre les dilutions 1/40 et 1/4000 varient ici d'un facteur 5.

Pour s'assurer de la spécificité des réponses obtenues, on a par le même procédé mis à l'épreuve face au dactyle dilué 1/40 et 1/40000, un sérum de malade titrant 20 000 unités internationales en IgE (1000 fois la normale) sans allergie connue, dilué 1/100 et un sérum d'allergique à la poussière de maison dilué 1/10. Les résultats sont résumés dans le tableau III.

**TABLE III**

| | Temoin sans allergène | Dilution de l'allergène (dactyle) | |
|---|---|---|---|
| | | 1/40 | 1/40 000 |
| Sérum à 20 000 en IgE | 0,482 | 0,472 | 0,494 |
| Sérum positif à la poussière de maison | 0,426 | 0,402 | 0,425 |

Le signal obtenu pour les sérums seuls, caractéristique des complexes existants avec l'allergène spécifique (inconnu pour le premier sérum, et poussière de maison pour le second), est retrouvé identique pour les deux dilutions de dactyle. Il n'y a pas de réponse spécifique au dactyle, ce qui est conforme aux spécificités connues des sérums.

**Conclusion de cet exemple**

1. Avec l'anticorps utilisé, les 3 sérums de sujets allergiques montrent des taux élevés de complexe à IgE. Le sérum du patient non allergique ne réagit pas dans le test.

2. Pour l'allergène considéré (Dactyle), deux dilutions, 1/40 et 1/40 000 sont suffisantes pour signifier la spécificité de l'allergène. Les 2 sujets allergiques à un autre allergène ne réagissent pas. Le sérum du sujet allergique montre une très grande différence de densité optique finale entre 1/40 et 1/40 000.

**Application II - Utilisation de l'anticorps clone 2 anti-IgE**

a - Réactifs (préparés comme indiqué ci-dessus).

L'anticorps utilisé est le clone 2 anti-IgE préparé par Hybritech (La Jolla - CA - USA).

Les sérums négatif et positif sont les mêmes qu'à l'Application I. L'allergène est la même préparation de dactyle.

Le tampon de dilution du mélange allergène-sérum est du sérum de chèvre à 10 % dans un tampon PBS-Tween®.

Le substrat est incubé 3 h à 22° C.

b - Résultats

**TABLEAU IV**

| | Témoin sans allergène | Dilution d'allergène | | | | |
|---|---|---|---|---|---|---|
| | | 1/40 | 1/400 | 1/4000 | 1/40000 | 1/400000 |
| Tampon de dilution | | 0,000 | 0,010 | 0,003 | 0,002 | 0,008 |
| Sérum négatif | 0,009 | 0,008 | 0,025 | 0,012 | 0,014 | 0,017 |
| Sérum positif | 0,287 | 0,199 | 0,229 | 0,310 | 0,323 | 0,288 |

Les résultats de l'exemple 4 sont retrouvés quoiqu'avec une sensibilité plus faible. La dilution 1/40 000 de l'allergène est encore celle qui donne la réponse maximum.

Des résultats du même ordre ont été obtenus pour des conditions identiques avec l'anticorps monoclonal anti-IgE n° 3001 élaboré par Transformation Research Inc. (M.A. - USA).

**Conclusion des applications**

Ces applications montrent que le système décrit permet d'une part de détecter des complexes circulants et d'autre part, avec deux dilutions d'un allergène supposé, de signer la nature de l'allergie.

Plusieurs anticorps monoclonaux de spécificité proche donnent des résultats concordants avec une sensibilité variable suivant la qualité de l'anticorps et la nature du diluant.

Le test est réalisé avec une solution d'allergène non fixée sur un support. Il suffit que l'allergène comporte au moins deux sites de fixation pour l'IgE sérique. Cela est le cas des allergènes utilisés pour la désensibilisation. La pénicilline, par exemple, est fixée sur de la polylysine pour l'utilisation in vivo.

Les exemples cités sont d'autant plus facilement généralisables que la présentation de l'allergène n'est pas modifiée par rapport à celle utilisée par les allergologues.

Une simple dilution est réalisée pendant le test.

L'application du test peut sans peine être étendue à tous les allergènes connus qui peuvent être classés dans les rubriques suivantes à titre purement indicatif:

- pollens et extraits de plante: dactyle...
- poussières
- acariens
- poils d'animaux domestiques: chien, chat...
- aliments oeuf, poisson...
- champignons, bactéries
- médicaments péniciline, insuline.

Ce système peut en outre être étendu à la recherche de complexes immuns mettant en cause des IgM, IgG, IgA, IgD ou des sous-classes de ces immunoglobulines.

Dans ce cas, l'anticorps utilisé doit être spécifique d'un site non répétitif de l'une de ces molécules. La réaction est conduite de la même façon que dans les exemples cités, avec la possibilité de détecter des complexes immuns préexistants et celle de repérer une spécificité particulière comme par exemple des IgM spécifiques de la Toxoplasmose, des IgA spécifiques d'une infection digestive ou des IgG spécifiques de la Rubéole.

Des tests polyantigéniques ou polyallergéniques sont particulièrement faciles à mettre en oeuvre par ce test puisque les antigènes ou les allergènes sont en phase liquide et peuvent donc être utilisés sur des gammes de concentration très larges.

Ce système permet également d'utiliser des antigènes figurés de taille très importante qui seraient difficiles à fixer de façon reproductible sur un support.

## Revendications

1. Procédé de détection d'un oligomère contenant au moins deux molécules possédant chacune un même site récepteur non répétitif, dans un milieu liquide contenant ou susceptible de contenir une solution ou dispersion de ladite molécule à l'état de monomère, ladite solution ou dispersion contenant aussi, ou étant susceptible de contenir ledit oligomère, caractérisé par le fait que l'on met en contact avec ladite solution ou dispersion un support solide sur lequel sont fixées des molécules liantes douées d'une affinité spécifique pour ledit site récepteur non répétitif, que l'on ajoute à ladite solution ou dispersion une solution ou dispersion de ladite molécule liante couplée à un agent traceur, et que l'on détermine qualitativement et/ou quantitativement la présence éventuelle dudit agent traceur fixé sur ledit support solide.

2. Procédé selon la revendication 1, caractérisé par le fait que ledit oligomère est un complexe immun formé par un premier anticorps, tel qu'un complexe immun du type anticorps/antigène/anticorps, et que ladite molécule liante est un second anticorps spécifique dirigé contre un site non répétitif dudit premier anticorps.

3. Procédé selon la revendication 2, caractérisé par le fait que ledit second anticorps spécifique est dirigé contre un site non répétitif du fragment Fc dudit premier anticorps.

4. Procédé selon l'une quelconque des revendications 2 et 3, caractérisé par le fait que ledit premier anticorps est une immunoglobuline IgE, IgD, IgG ou IgA, ou appartient à une sous-classe de ces immunoglobulines.

5. Procédé selon l'une quelconque des revendications 2 à 4, caractérisé par le fait que ledit second anticorps est un anticorps anti-IgE.

6. Procédé selon l'une quelconque des revendications 2 à 5, caractérisé par le fait que ledit second anticorps est un anticorps monoclonal.

7. Procédé selon le revendication 1, caractérisé par le fait que ledit oligomère est un complexe immun, tel qu'un complexe immun du type anticorps/antigène/anticorps, et que ladite molécule liante est une lectine.

8. Procédé selon l'une quelconque des revendications précédentes, caractérisé par le fait que l'on ajoute audit milieu liquide, contenant lesdits monomères, une substance dimérisante ou oligomérisante spécifique pour lesdits monomères ou pour une sous-classe desdits monomères que ledit milieu liquide est susceptible de contenir, de façon à former in situ ledit oligomère et à détecter la présence dudit oligomère, et en conséquence la présence du monomère pour lequel ladite substance dimérisante ou oligomérisante est spécifique.

9. Procédé selon la revendication 8, caractérisé par le fait que ladite substance dimérisante ou oligomérisante est un antigène, et que ledit monomère est l'anticorps correspondant.

10. Procédé selon la revendication 9, caractérisé par le fait que l'antigène est un allergène et que ledit monomère est une IgE dirigée contre ledit allergène.

11. Procédé selon l'une quelconque des revendications 8 à 10, caractérisé par le fait que l'on ajoute ladite substance dimérisante ou oligomérisante sous une forme liquide.

12. Procédé selon la revendication 11, caractérisé par le fait que ladite substance dimérisante ou oligomérisante est une solution d'antigène ou d'allergène.

13. Procédé selon l'une quelconque des revendications précédentes, caractérisé par le fait que ledit support solide est une bille, un tube ou une plaque d'un matériau sur lequel il est possible de fixer par affinité ou par couplage covalent ladite molécule liante.

14. Procédé selon l'une quelconque des revendications précédentes caractérisé par le fait que ledit agent traceur est un agent traceur fluorescent, enzymatique ou isotopique.

15. Procédé selon l'une quelconque des revendications 1 à 12, caractérisé par le fait que ladite molécule liante est fixée sur de petites particules solides individuelles capables de se maintenir en suspension dans ledit milieu liquide, que lesdites particules solides jouent à la fois le rôle dudit support solide et dudit traceur, et que l'on repère une éventuelle agglutination avec des moyens appropriés.

16. Dispositif destiné à la mise en oeuvre du procédé selon l'une quelconque des revendications précédentes, caractérisé par le fait qu'il comprend une ou plusieurs unités dudit support solide sur lequel est fixée ladite molécule liante, un ou plusieurs conteneurs contenant ladite molécule liante couplée à un agent traceur, éventuellement des solutions d'antigènes ou d'allergènes, des tampons de dilution, d'incubation et de lavage, et éventuellement un mode d'emploi, le tout étant réuni dans un même emballage à plusieurs compartiments.

## Patentansprüche

1. Verfahren zur Bestimmung eines Oligomeren, das mindestens zwei Moleküle enthält, von denen jedes eine gleiche sich nicht wiederholende Rezeptionsstelle besitzt, in einem flüssigen Milieu, das eine Lösung oder Dispersion dieses Moleküls im monomeren Zustand enthält oder zu enthalten in der Lage ist, wobei diese Lösung oder Dispersion außerdem das Oligomere enthält oder zu enthalten in der Lage ist, dadurch gekennzeichnet, daß man einen festen Träger mit der Lösung oder Dispersion in Kontakt bringt, auf den diejenigen Moleküle fixiert sind, die mit einer für eine Bindung spezifischen Affinität für die sich nicht wiederholenden Rezeptionsstellen ausgestattet sind, daß man zu dieser Lösung oder Dispersion eine Lösung oder Dispersion des bindungsfähigen, an einen Tracer gekuppelten Moleküls zufügt und daß man qualitativ und/oder quantitativ die eventuelle Gegenwart des an den festen Träger gebundenen Tracers bestimmt.

2. Verfahren nach Anspruch 1, dadurch gekennzeichnet, daß das Oligomere ein Immunkomplex ist, der durch einen ersten Antikörper gebildet ist, wie ein Immunkomplex des Typs Antikörper/Antigen/Antikörper, und daß das bindungsfähige Molekül ein zweiter spezifischer Antikörper ist, der gegen eine sich nicht wiederholende Stelle des ersten Antikörpers ausgerichtet ist.

3. Verfahren nach Anspruch 2, dadurch gekennzeichnet, daß der zweite spezifische Antikörper gegen eine sich nicht wiederholende Stelle des Fragments Fc des ersten Antikörpers ausgerichtet ist.

4. Verfahren nach den Ansprüchen 2 und 3, dadurch gekennzeichnet, daß der erste Antikörper ein Immunoglobulin IgE, IgD, EgG oder IgA oder eine zugehörige Unterklasse dieser Immunglobuline ist.

5. Verfahren nach den Ansprüchen 2 bis 4, dadurch gekennzeichnet, daß der zweite Antikörper ein Antikörper Anti-IgE ist.

6. Verfahren nach den Ansprüchen 2 bis 5, dadurch gekennzeichnet, daß der zweite Antikörper ein monoklonaler Antikörper ist.

7. Verfahren nach Anspruch 1, dadurch gekennzeichnet, daß das Oligomere ein Immunkomplex ist, wie ein Immunkomplex vom Typ Antikörper/Antigen/Antikörper, und das bindungsfähige Molekül ein Lektin ist.

8. Verfahren nach den vorhergehenden Ansprüchen, dadurch gekennzeichnet, daß man das flüssige Milieu, das die Monomeren enthält, zu einer dimerisierenden oder oligomerisierenden Substanz, die für die Monomeren oder für eine Unterklasse dieser Monomeren, die das flüssige Milieu zu enthalten in der Lage ist, spezifisch ist zugibt derart, daß das Oligomere in situ gebildet wird, und daß man die Gegenwart des Oligomeren und damit die Gegenwart des Monomeren, für das diese dimerisierende oder oligomerisierende Substanz spezifisch ist, bestimmt.

9. Verfahren nach Anspruch 8, dadurch gekennzeichnet, daß die dimerisierende oder oligomerisierende Substanz ein Antigen ist und daß das Monomere der entsprechende Antikörper ist.

10. Verfahren nach Anspruch 9, dadurch gekennzeichnet, daß das Antigen ein Allergen ist und daß das Monomere eine IgE ist, das gegen das Allergen gerichtet ist.

11. Verfahren nach den Ansprüchen 8 bis 10, dadurch gekennzeichnet, daß man die dimerisierende oder oligomerisierende Substanz in einer flüssigen Form zugibt.

12. Verfahren nach Anspruch 11, dadurch gekennzeichnet, daß die dimerisierende oder oligomerisierende Substanz eine Lösung des Antigens oder Allergens ist.

13. Verfahren nach den vorhergehenden Ansprüchen, dadurch gekennzeichnet, daß der feste Träger eine Kugel, ein Rohr oder eine Platte aus einem Material ist, an welches die Fixierung durch Affinität oder durch kovalentes Kuppeln des bindungsfähigen Moleküls möglich ist.

14. Verfahren nach den vorhergehenden Ansprüchen, dadurch gekennzeichnet, daß der Tracer ein

EP 0 132 170 B1

fluoreszierender, enzymatischer oder radioisotoper Tracer ist.

15. Verfahren nach den Ansprüchen 1 bis 12, dadurch gekennzeichnet, daß das bindungsfähige Molekül an den kleinen festen Einzelteilchen fixiert ist, welche in der Lage sind, in Suspension in dem flüssigen Milieu gehalten zu werden und daß die festen Teilchen gleichzeitig die Rolle des festen Trägers und des Tracers spielen und daß man eine eventuelle Agglutination mit geeigneten Mitteln markiert.

16. Vorrichtung zur Durchführung des Verfahrens nach einem der vorhergehenden Ansprüche, dadurch gekennzeichnet, daß sie aus einer oder mehreren Einheiten des festen Trägers besteht, auf welchem das bindungsfähige Molekül fixiert ist, sowie einen oder mehrere Behälter umfaßt, die das bindungsfähige Molekül, gekuppelt an einen Tracer, sowie gegebenenfalls Lösungen von Antigenen oder Allergenen, Puffer für die Verdünnungen, das Inkubieren und die Wäsche enthalten, sowie gegebenenfalls eine Gebrauchsanweisung enthält, wobei alle diese Teile in der gleichen Verpackung in Form mehrerer Abteile vereinigt sind.

## Claims

1. Process for detecting an oligomer containing at least two molecules, each having the same single nonrepetitive receptor site, in a liquid medium containing or liable to contain a solution or dispersion of the said molecule in the monomeric state, the said solution or dispersion also containing, or being liable to contain, the said oligomer, characterized in that the said solution or dispersion is brought into contact with a solid support on which there are fixed binding molecules endowed with a specific affinity for the said nonrepetitive receptor site, that there is added to the said solution or dispersion a solution or dispersion of the said binding molecule coupled with a tracer agent, and that the possible presence of the said tracer agent fixed onto the said solid support is determined qualitatively and/or quantitatively.

2. Process according to Claim 1, characterized in that the said oligomer is an immune complex formed by a first antibody, such as an immune complex of the antibody/ antigen/antibody type, and that the said binding molecule is a second specific antibody directed against a nonrepetitive site of the said first antibody.

3. Process according to Claim 2, characterized in that the said second specific antibody is directed against a nonrepetitive site of the fragment Fc of the said first antibody.

4. Process according to either of Claims 2 and 3, characterized in that the said first antibody is an IgE, IgD, IgG or IgA immunoglobulin or belongs to a subclass of these immunoglobulins.

5. Process according to any one of Claims 2 to 4, characterized in that the said second antibody is an anti-IgE antibody.

6. Process according to any one of Claims 2 to 5, characterized in that the said second antibody is a monoclonal antibody.

7. Process according to Claim 1, characterized in that the said oligomer is an immune complex, such as an immune complex of the antibody/antigen/antibody type, and that the said binding molecule is a lectin.

8. Process according to any one of the preceding claims, characterized by adding to the said liquid medium containing the said monomeres a dimerizing or oligomerizing substance specific for the said monomers or for a subclass of the said monomers which the said liquid medium is liable to contain, so as to form the said oligomer in situ and to detect the presence of the said oligomer, and consequently the presence of the monomer for which the said dimerizing or oligomerizing substance is specific.

9. Process according to Claim 8, characterized in that the said dimerizing or oligomerizing substance is an antigen, and that the said monomer is the corresponding antibody.

10. Process according to Claim 9, characterized in that the antigen is an allergen and that the said monomer is an IgE directed against the said allergen.

11. Process according to any one of Claims 8 to 10, characterized in that the said dimerizing or oligomerizing substance is added in a liquid form.

12. Process according to Claim 11, characterized in that the said dimerizing or oligomerizing substance is a solution of antigen or of allergen.

13. Process according to any one of the preceding claims, characterized in that the said solid support is a bead, a tube or a plaque of a material onto which it is possible to fix the said binding molecule by affinity or by covalent coupling.

14. Process according to any one of the preceding claims, characterized in that the said tracer agent is a fluorescent, enzymatic or isotopic tracer agent.

15. Process according to any one of Claims 1 to 12, characterized in that the said binding molecule is fixed onto individual small solid particles capable of remaining suspended in the said liquid medium, that the said solid particles act both as the said solid support and the said tracer, and that a possible agglutination is registered with suitable means.

16. Device intended for implementing the process according to any one of the preceding claims, characterized in that it comprises one or more units of the said solid support onto which the said binding molecule is fixed, one or more containers containing the said binding molecule coupled to a tracer agent, optional antigen or allergen solutions, dilution, incubation and washing buffers and optional instructions for use, the whole being combined in a single package containing a number of compartments.

FIGURE 1 : FIXATION DES COMPLEXES IMMUNS

FIGURE 2 : REACTION EN L'ABSENCE DE COMPLEXES IMMUNS

FIGURE 3 : REVELATION DES COMPLEXES IMMUNS PAR AGGLUTINATION
DE PARTICULES DE LATEX

5